# EUROPEAN PATENT SPECIFICATION

(11) **EP 1 973 397 B1**
(45) Date of publication and mention of the grant of the patent: **02.10.2024**
(21) Application number: 07718255.8
(22) Date of filing: 10.01.2007
(51) Int. Cl.: A01H 5/00, A01H 1/00

(54) **NOVEL CUCURBITA PLANTS**
NEUARTIGE CUCURBITA-PFLANZEN
NOUVELLES CUCURBITACÉES

(30) Priority: 13.01.2006 US 758816 P; 18.04.2006 US 405975
(43) Date of publication of application: 01.10.2008
(73) Proprietor: Syngenta Crop Protection AG, 4058 Basel (CH)
(72) Inventor: NICOLET, Jean Louis Marie, Edouard, F-84260 Sarrians (FR); COOK, Kevin, Naples, FL 34114 (US); GUSMINI, Gabriele, Naples, FL 34114 (US); NICOLAS, Matthieu Jean, 84260 Sarrians (FR)
(74) Representative: SYNGENTA IP
(86) International application number: PCT/US2007/000604
(87) International publication number: WO 2007/084299

(56) References cited:
- US-B1- 6 342 655
- SHIFRISS C ET AL: "AN EVALUATION OF F-2 POPULATIONS FROM A CROSS BETWEEN CUCURBITA-PEPO AND CUCURBITA-MOSCHATA FOR RESISTANCE TO CUCUMBER MOSAIC VIRUS", EUPHYTICA, vol. 23, no. 2, 1974, pages 333 - 336, XP008110403, ISSN: 0014-2336
- BROWN REBECCA N ET AL: "Inheritance of resistance to four cucurbit viruses in Cucurbita moschata.", EUPHYTICA, vol. 129, no. 3, 2003, pages 253 - 258, XP002542100, ISSN: 0014-2336
- SITTERLY, W. R.: "Breeding for diseease resistance in cucurbits", ANNUAL REVIEW OF PHYTOPATHOLOGY, vol. 10, 1972, pages 471 - 490, XP002542101
- DATABASE BIOSIS [online] BIOSCIENCES INFORMATION SERVICE, PHILADELPHIA, PA, US; 1985, PINK D A C ET AL: "EFFECT OF TEMPERATURE AND LIGHT INTENSITY ON RESISTANCE IN MARROW CUCURBITA-PEPO TO CUCUMBER MOSAIC VIRUS", XP002542338, Database accession no. PREV198580025607
- JOURNAL OF AGRICULTURAL SCIENCE, vol. 104, no. 2, 1985, pages 325 - 330, ISSN: 0021-8596
- WU, T. & CAO, J.: "Molecular cloning and expression of a bush related CmV1 gene in tropical pumpkin", MOL. BIOL. REP., 24 March 2009 (2009-03-24), pages 1-4, XP002542103, Retrieved from the Internet <URL:http://www.springerlink.com/content/nqu227pqt7160362/> [retrieved on 20090819]
- 2004 WISCONSIN VEGETABLE DISEASE CONTROL TRIALS, 2004
- 2004 CUCURBIT CULTIVAR EVALUATIONS, 2004

## Description

### FIELD OF THE INVENTION

The present invention relates to novel *Cucurbita* plants resistant to viruses. The present invention also relates to seeds and parts of said plants, for example fruits. The present invention also relates to methods of making and using such plants.

### BACKGROUND OF THE INVENTION

The *Cucurbita* are members of the family *Cucurbitaceae,* which comprises about 90 genera and 700 to 760 species, mostly of the tropics. The genus *Cucurbita* includes four major species, *pepo, argyrosperma, moschata,* and *maxima,* one minor *species, ficifolia,* and some wild ones. The major commercial crops of the genus *Cucurbita* are squash and pumpkin.

Squash and pumpkins are unique in that they represent several species for the same crop. They encompass many summer and winter edible types as well as ornamental gourds. Summer squash is *Cucurbita pepo,* but winter squash may be *C. pepo* (e.g. 'Acorn'), *C. moschata* ('Butternut'), *C. mixta* (`Japanese Pie'), or *C. maxima* (`Hubbard', etc.). The Jack'O' Lantern type of pumpkin is *C. pepo,* but commercially canned pumpkin pie mix is also made from *C. moschata* (e.g. 'Libby Select') or *C. maxima* ('NK 580'). *C. mixta* and *C. ficifolia* are used for food in Mexico and in Central and South American countries.

Squash and pumpkin are usually grown for their fruit, harvested immature for summer squash or mature for winter squash and pumpkin. Summer squash is most often eaten fresh, but winter squash may be stored several months. Both summer and winter squash are canned and frozen commercially. It has been reported that the world production of *Cucurbita* in 1979-81 was 5,256,000 metric tons from 539,000 acres annually.

Several types of viruses affect crops of *Cucurbita* plants, for example Zucchini Yellow Mosaic Virus (ZYMV), Watermelon Mosaic Virus (WMV), and Papaya Ringspot Virus (PRSV). Cucumber Mosaic Virus (CMV), which belongs to the genus of the Cucumoviruses, family *Bromoviridae* (Roossinck (2002) Journal of Virology 76: 3382-3387), also affects crops of *Cucurbita* plants.

These viruses cause extensive damages to *Cucurbita* crops and new strategies to control them are always desired. There is therefore an unfulfilled need for new sources of genetic resistance to viruses in *Cucurbita,* in particular new sources of resistance to CMV in *Cucurbita.*

### SUMMARY OF THE INVENTION

The instant invention addresses the unfulfilled need for new sources of resistance to viruses in *Cucurbita,* in particular the unfulfilled need for new sources of resistance to CMV in *Cucurbita.* To address these needs, the instant invention provides novel *Cucurbita* plants comprising a new gene conferring resistance to viruses upon said plants, in particular resistance to CMV.

The invention is defined by the appended claims.

The new gene, designated *Cmv-2,* was surprisingly found in *C. moschata* var. Seminole pumpkin and successfully transferred to other *Cucurbita* plants using embyo rescue and backcross techniques. The *Cmv-2* gene is located at the chromosomal locus of the gene conferring resistance to CMV in *C. moschata* var. Seminole pumpkin or in inbred line 90-3588, representative seed of which being deposited under accession number NCIMB 41369. Accordingly, a plant according to the present invention is. a plant comprising a *Cmv-2* gene. The *Cmv-2* gene confers upon a *Cucurbita* plant high level of virus resistance. The resistance conferred by the *Cmv-2* gene is stable and can be transferred to other *Cucurbita* plants, for example *Cucurbita* plants susceptible to the virus. In one embodiment, the *Cmv-2* gene is obtainable from a plant of *C. moschata* var. Seminole pumpkin. In one embodiment, the *Cmv-2* gene is obtainable from a plant of line 90-3588. In one embodiment, the *Cmv-2* gene is dominant. In one embodiment, resistance to CMV according to the instant invention is monogenic.

In one embodiment, a plant of the invention is a *Cucurbita* plant comprising a *Cmv-2* gene, wherein said *Cucurbita* plant is not a plant of *C. moschata* var. Seminole pumpkin. In one embodiment, a plant of the invention is a plant of *C. pepo,* a plant of *C. maxima* or a plant of *C. mixta.* In one embodiment, a plant of the invention is a squash plant. In one embodiment, a plant of the invention is a summer squash plant, a winter squash plant or a pumpkin plant. In one embodiment, a plant of the invention is a plant of *C. pepo L..* In one embodiment, a plant of the instant invention is homozygous for a *Cmv-2* gene.

The instant invention also provides seeds and parts of the plants of the invention, for example fruits, and methods of making and using such plants. The *Cmv-2* gene of the instant invention can also be combined with other resistance strategies to further increases resistance to the virus, for example horizontal resistance.

The instant invention allows growers to prevent or reduce damages to their crops caused by the virus, and also allows for increased flexibility in their resistance strategies. Higher yields are obtained and fruit can be harvested over a longer period of time.

The present invention therefore provides:
A *Cucurbita* plant comprising a *Cmv-2* gene conferring Cucumber Mosaic Virus (CMV) resistance to said *Cucurbita* plant, wherein said *Cmv-2* gene is obtainable from a plant of *C. moschata* cv. Seminole pumpkin, wherein, when said *Cucurbita* plant is crossed with a plant of inbred line 90-3588, 100% of F1 plants resulting from said cross are resistant to CMV, and 100% of said F1 plants produce 100% of F2 progeny plants resistant to CMV, wherein said *Cucurbita* plant is homozygous for a *Cmv-2* gene, wherein said plant is not a plant of *C. moschata* cv. Seminole pumpkin, wherein said plant is male sterile, wherein resistance to CMV is monogenic and wherein said *Cmv-2* gene is obtainable from a plant of line 90-3588, representative seed of which being deposited under accession number NCIMB 41369.

In one embodiment, the plant is a *C. pepo* plant, a *C. maxima* plant or a *C. mixta* plant, in one embodiment, a *C. pepo* L. plant. In one embodiment, the *Cmv-2* gene is obtainable from a plant of *C. moschata* cv. Seminole Pumpkin or from a plant of line 90-3588, representative seed of which being deposited under accession number NCIMB 41369. In one embodiment, the plant is homozygous for said *Cmv-2* gene. In one embodiment, the plant is an inbred line, a hybrid or a dihaploid. In one embodiment, the plant is male-sterile.

The present invention further provides:
A *C. pepo* plant highly resistant to CMV, wherein the plant comprises a *Cmv-2* gene. In one embodiment, the plant is a *C. pepo L.* plant. In one embodiment, the *Cmv-2* gene is obtainable from a plant of *C. moschata* cv. Seminole Pumpkin or from a plant of line 90-3588, representative seed of which being deposited under accession number NCIMB 41369. In one embodiment, the plant is homozygous for said *Cmv-2* gene. In one embodiment, the plant is an inbred line, a hybrid or a dihaploid. In one embodiment, the plant is male-sterile. In one embodiment, the plant is identified and defined in an allelic test as described herein.

The present invention further provides:
A *Cucurbita* plant of inbred line 90-3588, representative seed of which been deposited under accession NCIMB 41369.

The present invention further provides:
Seed or part of anyone of the *Cucurbita* plants above. In one embodiment, the part is a cell, pollen, ovule or fruit.

The present invention further provides:
A method of vegetatively propagating a *Cucurbita* plant comprising: collecting a tissue from anyone of the *Cucurbita* plants above; cultivating said tissue to obtain proliferated shoots; rooting said proliferated shoots to obtain rooted plantlets.

The present disclosure further provides:
A *Cucurbita* plant comprising a *Cmv-2* gene obtainable by the method above.

### DEFINITIONS

Gene: Unit of inheritance. Genes are located at fixed loci in chromosomes. A gene can exist in a series of alternative forms called alleles. A gene can control or contribute to a trait, for example resistance to a disease.

Trait: characteristic or phenotype, for example resistance to a disease. A trait may be inherited in a dominant or recessive manner. A trait may be monogenic or polygenic, or may also result from the interaction of one or more genes, in some cases with the environment. A trait is for example resistance to one or more viruses, for example CMV.

*Cmv-2* gene: gene conferring resistance to CMV upon a *Cucurbita* plant and located at the chromosomal locus of the gene conferring resistance to CMV in inbred line 90-3588 or in *C. moschata* var. Seminole pumpkin.

"Tester" plant: plant used to characterize genetically a gene or a trait in a plant to be tested. Typically, the plant to be tested is crossed with a "tester" plant and the segregation ratio of the trait in progeny plants of the cross is scored.

Allele: One of a pair or series of forms of a gene, which are alternative in inheritance because they are situated at the same locus in homologous chromosomes.

Locus: region on a chromosome, which generally comprises a gene, e.g. a gene controlling or contributing to a trait.

Resistance: biological mechanism based on the genotype of a plant, by which a plant exhibits no symptoms, insignificant symptoms, or reduced symptoms of a disease in a resistant plant, as compared to the plants without the resistance (susceptible plants), which are affected by the same disease at the same time, e.g. during the same production cycle, and in the same production unit. Resistance is thus preferably the ability of a plant to reduce the development of a pathogen, (see for example Robinson, R.A., 1969: Review Applied Mycology 48, 593-606).

Resistance to CMV: defined as the ability of a plant to reduce the development of CMV epidemics, whereas susceptible plants are affected by CMV at the same time, e.g. during the same production cycle, and in the same production unit.

Monogenic: determined by a single locus.

Polygenic: determined by more than one locus.

Dominant: results in a complete phenotypic manifestation at heterozygous or homozygous state. However, in some cases a gene dosage effect may be observed, which may result in a slightly stronger and more reproducible phenotype at the homozygous state than at the heterozygous state.

Recessive: manifests itself only when present at homozygous state.

Genetic linkage: association of characters in inheritance due to location of genes in proximity on the same chromosome. Measured by percent recombination (i.e. recombination frequency) between loci (centi-Morgan, cM).

Isogenic: plants, which are genetically identical, except that they may differ by the presence or absence of a gene at a locus conferring a trait or heterologous DNA sequence.

Marker assisted selection: refers to the process of selecting a desired trait or desired traits in a plant or plants by detecting one or more nucleic acids from the plant, where the nucleic acid is associated with the desired trait.

Dihaploid: plants generated by doubling of haploid plants (single chromosome set) (e.g. through anther culture or microspore culture) giving a complete homozygous plant.

As used herein, the term "plant" includes plant cells, plant protoplasts, plant cells of tissue culture from which *Cucurbita* plants can be regenerated, plant calli, plant clumps, and plant cells that are intact in plants or parts of plants, such as pollen, flowers, seeds, fruits, leaves and the like.

Regeneration: refers to the development of a plant from tissue culture.

Single Gene Converted (Conversion): refers to plants which are developed by a plant breeding technique called backcrossing wherein essentially all of the desired morphological and physiological characteristics of a variety are recovered in the offspring in addition to the single gene transferred into the variety via the backcrossing technique or via genetic engineering.

### DETAILED DESCRIPTION OF THE INVENTION

The instant invention addresses the unfulfilled need for new sources of resistance to viruses in *Cucurbita,* in particular the unfulfilled need for new sources of resistance to CMV in *Cucurbita.* To address these needs, the instant invention provides novel *Cucurbita* plants comprising a new gene conferring resistance to viruses upon said plants. In particular, the new gene of the instant invention confers resistance to CMV upon said *Cucurbita* plants. The instant invention also provides seeds and parts of the plants of the invention, for example fruits. The present disclosure also provides methods of making and using such plants.

The plants of the present invention comprise a gene conferring upon said plants resistance to CMV and designated as *Cmv-2.* The *Cmv-2* gene was unexpectedly found in *C. moschata* var. Seminole pumpkin (also referred herein to as *C. moschata* cv. Seminole Pumpkin) and transferred to other *Cucurbita* plants.

Seminole Pumpkin is a winter squash, species *C. moschata.* This squash is originally from the Southeastern United States, and its original name, Chassa howitska, means "hanging pumpkin". Seminole Pumpkin plants can be grown as climbing vines over trees, with the pumkin fruit hanging down from the branches, out of reach of livestock and rot. The leaves of this winter squash are rounded, slightly lobed, medium to dark green in color, with a greyish mottling on the older leaves. The vines are large and bear bell-shaped, cream fruits, approximately 10 to 12 cm wide at the bulb. Generally, fruits are similar to 'Butternut', but sweeter and have a firm, deep, orange colored flesh. The flowers are orange and wide. As most cucurbits, the plant is monoecious and both male and female flowers are fully fertile.

The *Cmv-2* gene was transferred to other *Cucurbita* plants using embyo rescue and backcross techniques. Firstly, Seminole Pumpkin was inbred for five cycles by artificial self-pollination. The F5 generation was then crossed with a proprietary inbred line of the yellow straightneck type, thus performing an interspecific cross between *C. pepo* and *C. moschata.* Embryo rescue techniques were used to obtain a few viable plants from this cross and self-pollinated seed of these plants. The detailed procedures are described in Example 1 below.

Interspecific progenies were obtained and used in several backcross breeding programs, thus producing an array of populations segregating for resistance to CMV derived from *C. moschata* var. Seminole pumpkin. The resistance conferred by the *Cmv-2* gene is stable and can be transferred to other *Cucurbita* plants, in particular to other *Cucurbita* plants susceptible to the virus.

Accordingly, in one embodiment, the *Cmv-2* gene is obtainable from a plant of *C. moschata* var. Seminole pumpkin.

In one embodiment, the resistance to CMV is tested and assessed as described in Example 2 below. However, the person skilled in the art would also be able to use other protocols or methods to determine resistance to the virus. For example, viral RNA and protein can also be measured, for example as described in Kaplan et al. (1997) Virology 230: 343-349 and Mayers et al. (2005) MPMI18:428-434. Leaf-press blotting can also be carried out as described for example in Kaplan et al. (1997) Virology 230: 343-349.

The *Cmv-2* gene confers resistance to various strains of CMV from different geographical locations. For example, the *Cmv-2* gene confers upon a *Cucurbita* plant resistance to the Florida strain of CMV (University of Florida, Gainsville, Florida). The *Cmv-*2 gene also confers upon a *Cucurbita* plant resistance to the California pepper strain of CMV and to the CMV-TL strain, a European strain of CMV (INRA, Virology Department, Montfavet 84, France). Other strains of CMV can also be tested as described herein.

In one embodiment, a plant of the invention is homozygous for a *Cmv-2* gene. In one embodiment, the *Cmv-2* gene is dominant. In one embodiment, the resistance to CMV according to the instant invention is monogenic. In one embodiment, the resistance to CMV according to the instant invention is monogenic and dominant.

A representative *Cucurbita* line comprising the *Cmv-2* gene according to the present invention, inbred line 90-3588, was deposited with NCIMB, Aberdeen AB21 9YA, Scotland, UK under accession number NCIMB 41369 on January 11, 2006. Plants of line 90-3588 are homozygous for the *Cmv-2* gene. In one embodiment, the *Cmv-2* gene is obtainable from a plant of line 90-3588. In one embodiment, a plant of the instant invention is a plant of inbred line 90-3588, representative seeds of which been deposited under accession number NCIMB 41369 (see Example 3 below).

The *Cmv-2* gene is located at the chromosomal location of the gene conferring resistance to CMV in *C. moschata* var. Seminole pumpkin or in line 90-3588. In one embodiment, a plant comprising a *Cmv-2* gene is identified and defined by an allelic test. An allelic test is performed by making a cross between a plant of a "tester" line (i.e. "tester" plant) and the plant to be tested, and determining the segregation ratio of plants resistant to CMV to plants susceptible to CMV in the progeny of the cross. Typically, Fl progeny plants and F2 progeny plants resulting from self-pollination of the Fl progeny plants are assessed. Progeny plants resulting from backcrosses with the parents and plants obtained after self-pollination of backcross progeny plants may also be assessed. The segregation ratio of resistant to susceptible plants can also be determined in subsequent generations of self-pollination and backcross with the parents. A tester plant is a plant comprising a *Cmv-2* gene. In one embodiment, a tester plant is a plant of inbred line 90-3588, deposited with NCIMB under accession number NCIMB 41369.

In one embodiment, if a tester plant homozygous for the *Cmv-2* gene and a plant to be tested homozygous for a single gene conferring resistance to CMV are used as parents in a cross, and only resistant plants are recovered in the Fl and F2 progenies (i.e. no segregation of resistant and susceptible plants), the plant to be tested comprises a *Cmv-2* gene, and is defined as plant of the instant invention. Accordingly, in one embodiment, a plant of the present invention is a *Cucurbita* plant comprising a *Cmv-2* gene, wherein when said *Cucurbita* plant is crossed with a plant of inbred line 90-3588 100% of F1 plants resulting from the cross are resistant to CMV, and 100% of the Fl plants produce 100% of F2 progeny plants resistant to CMV, when said *Cucurbita* plant is homozygous for a *Cmv-2* gene (see Table 1). In this situation, if F1 or F2 progeny plants are backcrossed with the parents and the progeny plants resulting from the backcrosses are self-pollinated, only resistant plants are recovered in the progeny plants resulting from the backcrosses and from subsequent self-pollination.

In one embodiment, if a tester plant homozygous for the *Cmv-2* gene and a plant to be tested heterozygous for the single gene conferring resistance to CMV are used as parents in a cross, and only resistant plants are recovered in the Fl progeny, and 100% of resistant F2 plants are recovered in the progeny of 50% of the Fl plants and a 3:1 ratio of resistant to susceptible F2 plants are recovered in the progeny plants of 50% of the Fl plants, the plant to be tested comprises a *Cmv-2* gene, and is defined as plant of the instant invention. Accordingly, in one embodiment, a plant of the present invention is a *Cucurbita* plant comprising a *Cmv-2* gene, wherein when said *Cucurbita* plant is crossed with a plant of inbred line 90-3588 100% of Fl plants resulting from said cross are resistant to CMV, and 50% of the Fl plants produce 100% of F2 progeny plants resistant to CMV and 50% the Fl plants produce a 3:1 ratio of resistant to susceptible F2 plants, when said *Cucurbita* plant is heterozygous for a *Cmv-2* gene (see Table 1).

By contrast, if a tester plant homozygous for the *Cmv-2* gene and a plant to be tested homozygous for a single gene conferring resistance to CMV are used as parents in a cross, and susceptible plants are found among F2 progeny plants, the plant to be tested contains a gene located at a different locus from that of the *Cmv-2* gene. The plant to be tested thus does not comprise a *Cmv-2* gene and is not a plant according to the instant invention. Susceptible plants are also found among F2 progeny plants if the plant to be tested is heterozygous for a single gene conferring resistance to CMV located at a different locus from that of the *Cmv-2* gene. In this situation, if Fl or F2 progeny plants are backcrossed with the parents and the progeny plants resulting from the backcross are self-pollinated, susceptible plants are found in the progeny obtained after the self-pollination following the backcross.

The person skilled in the art would know how to carry out an allelic test, determine the appropriate number of progeny plants to be tested in each generation, and analyze the results of the test. For example, if the tester plant and the plant to be tested are both homozygous for a dominant single gene conferring resistance to the virus but located at different, unlinked chromosomal loci, one would expect only resistant plants in the Fl progeny, but a 15:1 segregation of resistant to susceptible plants in F2 progeny plants. In this case, if the tester plant is homozygous for the *Cmv-2* gene and the plant to be tested is heterozygous for the single dominant gene located at a different, unlinked chromosomal locus one would expect only resistant plants in the Fl progeny, and a 3:1 segregation of resistant to susceptible plants in F2 plants of 50% of the Fl plants and a 15:1 segregation of resistant to susceptible plants in F2 plants of 50% of the Fl plants (see Table 1).

These segregation ratios may deviate from the above, figures if the single dominant gene located at a different, unlinked chromosomal locus confers a milder or less stable resistance, for example moderate resistance to CMV as described herein. In this case, some of the plants comprising the single dominant gene located at a different, unlinked chromosomal locus may be rated as susceptible.

If the two chromosomal loci are linked, a higher ratio of resistant to susceptible plants would be expected.

The person skilled in the art would know how to carry out an allelic test, determine the appropriate number of progeny plants to be tested in each generation, and know how to analyze the results of the test. It is also understood that in rare cases a plant comprising a gene conferring resistance to CMV may be rated as susceptible due to aberrant seed germination or abnormal plant development. Such plants are however not considered in the allelic tests and in determining the segregation ratios and are generally eliminated in the disease testing.

Other types of crosses and allelic test are also within the skills of a person of the art and may also be carried out. Additional generations may also be evaluated. A tester cross can also be carried out with a tester plant heterozygous for a *Cmv-2* gene and a plant to be tested heterozygous for a gene to be tested. The person skilled in the art would know how to carry out and interpret the result of these crosses.

**Table 1: Expected results of allelic tests using a plant of inbred line 90-3588 as tester plant.**

| Plant to be tested | | Fl progeny plants | F2 progeny plants |
|---|---|---|---|
| Plant comprising a *Cmv-2* gene | Homozygous for gene | 100% R | 100% R |
| | Heterozygous for gene | 100% R | 100% R in 50% of the Fl progeny plants |
| | | | 3: 1 ratio of R to S in 50% of the Fl progeny plants |
| | | | |
| Plant comprising a dominant single gene conferring resistance to CMV located at a different, unlinked chromosomal location from that of a *Cmv-2* gene | Homozygous for gene | 100% R | 15:1 ratio of R to S |
| | Heterozygous for gene | 100% R | 15:1 ratio of R to S in 50% of the Fl progeny plants |
| | | | 3:1 ratio of R to S in 50% of the Fl progeny plants |

| | | | |
|---|---|---|---|
| R: plant resistant to CMV, S: plant susceptible to CMV | | | |

In one embodiment, a plant of the invention is a *Cucurbita* plant comprising a *Cmv-2* gene, wherein said plant is not a plant of *C. moschata* var. Seminole pumpkin. In one embodiment, a plant of the invention is a plant of *C. pepo,* a plant of *C. maxima* or a plant of *C. mixta.* In one embodiment, a plant of the invention is a squash plant. In one embodiment a plant of the invention is a summer squash plant, a winter squash plant or a pumpkin plant. In one embodiment, a plant of the invention is a plant of *C. pepo L..*

*Cucurbita* pepo L. often refers to what is commonly known as the summer squash such as scallop, zucchini, straightneck and crookneck types, and winter squash such as acorn and pumpkin. Eight groups of edible *C. pepo* are known:
- Pumpkin *(C. pepo* L. var. *pepo* L. Bailey,) includes cultivars of creeping plants which produce spherical, oval or oblate fruit that is rounded or flat at the ends. The fruit of this group is grown to be eaten when ripe and sometimes is used as fodder.
- Scallop (C. *pepo* L. var. *clypeata* Alefield) has a semi-shrubby habit, the fruit ranges from flat to almost discoidal, with undulations or equatorial margins, and it is eaten before maturity.
- Acorn (C. *pepo* L. var. *turbinata* Paris) is both a shrubby and creeping plant with fruit which is obovoid or conical, pointed at the apex and longitudinally costate-grooved. The rind is soft, hence the fruit can be eaten in the ripe state.
- Crookneck (C. *pepo* L. var. *torticollia* Alefield) is a shrubby type, with yellow, golden or white.ffuit which is claviform and curved at the distal or apical end and generally has a verrucose rind. It is eaten unripe since the rind and the flesh harden when ripe.
- Straightneck (C. *pepo* L. var. *recticollis* Paris) is a shrubby plant with yellow or golden fruit and a verrucose rind similar to that of var *torticollia.*
- Vegetable marrow (C. *pepo* L. *varfastigata* Paris) has creeper characteristics as a semishrub and has short cylindrical fruit that is slightly broader at the apex, with a smooth rind which hardens and thickens on ripening and which varies in colour from cream to dark green.
- Cocozzelle (C. *pepo* L. var. *longa* Paris) has cylindrical, long fruit that is slender and slightly bulbous at the apex; it is eaten in the unripe state and one of the most common names is Cocozzelle.
- Zucchini (C. *pepo* L. var. *cylindrica* Paris) is the most common group of cultivars at present. Like the previous group, the zucchini group has a strong affinity with the vegetable marrow and its origin is also recent (nineteenth century). Its plants are generally semi-shrubby and its cylindrical fruit does not broaden or else broadens only slightly. It is eaten as a vegetable in the unripe state.

In one embodiment, a plant of the instant invention is a plant of any one of the eight groups above, or any other groups from different classifications, wherein said plant comprises a *Cmv-2* gene.

Squash plants usually develop a running vine on the soil but current summer squash cultivars have been developed in the form of a short compact bush, making them easier to grow in smaller spaces. On healthy winter squash plants, there is a canopy of large, reniform and serrated leaves, which may be without lobes or with very deep ones. Fruit flesh can be of various shade of yellow, or even from white to orange. The fruits may have a soft or a hard shell with colors from dull to bright. Summer squash show a great variety of shape, cylindrical, long, flat, etc., with sizes from small to large and colors from uniform to variegated. The flesh can range from white to yellow and, contrary to the winter squash that has a flesh finely grained, bear coarse grains.

Squash is a simple diploid species with twenty pairs of highly differentiated chromosomes. The plants are monoecious, with separate female and male flowers on the same plant. Usually, the first four or five flowers produced are male, then the next eight or so are female, followed by a few more male and female flowers. Male flowers have 3-5 erect stamens bunched within the corolla of 5 fused petals. Female flowers have 3 spreading stigma lobes and an immature fruit (external ovary) below the perianth. The spiny, sticky pollen requires insects for pollination. The primary pollinators are bees, particularly honeybees. Pollination generally occurs in the morning after the flowers open.

Based on the description of the present invention, the skilled person is able to recognize a *Cucurbita* plant of the instant invention, for example by performing an allelic test, for example an allelic test as described above. Based on the description of the present invention, the skilled person is able to recognize a plant resistant to CMV (also called herein "resistant plant") and a plant susceptible to CMV (also called herein "susceptible" plant), for example using a disease testing as described herein.

In one embodiment, the *Cmv-2* gene confers high level of resistance to CVM upon a *Cucurbita* plant, for example when tested and assayed as described in Example 2 below. Accordingly, in one embodiment, a plant resistant to CMV according to the present invention is a plant highly resistant to CMV (also called herein "highly resistant plant"). In one embodiment, the *Cucurbita* plant according to the present invention highly resistant to CMV is a plant of C. *pepo.* In one embodiment, said plant is a squash plant. In one embodiment, a highly resistant plant according to the present invention is identified and defined by an allelic test, for example an allelic test as described herein.

In a disease testing as described herein, a highly resistant plant shows insignificant symptoms of CMV infection. The first two true-leaves of a highly resistant plant may show symptoms of CMV infection, as in susceptible plants, but new leaves grow without any mosaic symptom, nor distortion and the plants do not show symptoms thereafter. Based on the description of the present invention, the skilled person is able to recognize a plant of the instant invention highly resistant to CMV, as example by using a disease testing as described herein. An example of a C. *pepo* plant highly resistant to CMV is a plant of inbred line 90-3588, representative seed of which being deposited under accession number NCIMB 41369, or a plant of hybrid RSQ2193 described herein. Accordingly, in one embodiment, a plant highly resistant to CMV shows the level of resistance as that of a plant of inbred line 90-3588 or of a plant of hybrid RSQ2193, for example in a disease test as disclosed herein.

In one embodiment, a C. *pepo* plant of the present invention is more resistant to CMV than currently available *C. pepo* plants, in particular when tested in a disease testing as described herein. For example, a *C. pepo* plant of the present invention is more resistant than a *C. pepo* plant moderately resistant to CMV (also called herein "moderately resistant plant"). In a disease testing described herein, a moderately resistant *C. pepo* plant shows reduced symptoms of CMV infection, such as symptoms of CMV infection beyond the first two true-leaves, sometimes on all leaves, delayed appearance of symptoms of CMV infection and symptoms remaining throughout plant growth. In some cases, moderately resistant plants may be overcome by the disease and may be rated as susceptible.

A *C. pepo* plant moderately resistant to CMV is for example a plant of squash hybrid 'Dividend' (a commercial variety of Syngenta Seeds, Inc.). Hybrid 'Dividend' comprises a *Cmv-1* gene in the heterozygous state. 'Dividend' has also been described as tolerant to CMV. Accordingly, in one embodiment, a C. *pepo* plant of the present invention is more resistant to CMV than a *C. pepo* plant comprising a *Cmv-1* gene. An allelic test between *C. pepo* plants comprising a *Cmv-2* gene and *C. pepo* plants comprising a *Cmv-1* gene was carried out and is described in Example 5 below. The allelic test indicates that the *Cmv-2* gene is located at a different chromosomal location than the *Cmv-1* gene.

Accordingly, in one embodiment, the *Cmv-2* confers upon a *C. pepo* plant higher level of resistance to CMV than the *Cmv-1* gene, in particular in a disease test as described herein. In one embodiment, the *Cmv-2* confers upon a *C. pepo* plant a more stable level of resistance to CMV than the *Cmv-1* gene, in particular in a disease test as described herein.

Disease tests were also carried out with *C. moschata* cv. Seminole Pumpkin and *C. moschata* cv. Nigerian Local. *C. moschata* cv. Nigerian Local is a winter squash, species *C. moschata,* described in Brown, 2003 (Euphytica 129: 253-258). The disease tests are described in Example 4 below. Plants of *C. moschata* cv. Seminole Pumpkin did not show any symptom of the disease and were rated as immune to CMV (also called herein "immune plant"). By contrast, plants of *C. moschata* cv. Nigerian Local showed symptoms of CMV infection on all plants tested and were rated as moderately resistant to CMV.. The symptoms in *C. moschata* cv. Nigerian Local were limited to mosaic on all or most of the leaves; none of the tested plants was stunted (Table 9).

Accordingly, in one embodiment, a *Cucurbita* plant resistant to CMV can be further defined as highly resistant to CMV, moderately resistant to CMV or immune to CMV. A *C .pepo* plant susceptible to CMV is for example a plant of 'Gentry' (a commercial variety of Syngenta Seeds, Inc.).

The *Cmv-2* gene was transferred to several types of *Cucurbita* plants. For example, the *Cmv-2* gene was transferred to plants of the yellow crookneck type and to plants of the zucchini type. Based on the description of the present invention, the skilled person is also able to transfer a *Cmv-2* gene to other *Cucurbita* plants lacking said gene, for example to a *Cucurbita* plant susceptible to CMV, and to produce a plant comprising the *Cmy-2* gene.

The following paragraph is not part of the invention.

Accordingly, the present specification further discloses a method of transferring a *Cmv-2* gene to a *Cucurbita* plant lacking said gene comprising: a) obtaining a plant comprising a *Cmv-2* gene; b) crossing it to a plant lacking said gene; c) obtaining plants . of the cross of step b); d) selecting a plant of step c) comprising the *Cmv-2* gene. In one aspect, the method further comprises: e) back-crossing a plant resulting from step d) with a *Cucurbita* plant, and f) selecting for a *Cucurbita* plant comprising the *Cmv-2* gene. In one aspect, the method further comprises obtaining an inbred *Cucurbita* plant comprising the *Cmv-2* gene, and, in one aspect, the method further comprises crossing said inbred *Cucurbita* plant to another *Cucurbita* plant to produce a hybrid *Cucurbita* plant comprising the *Cmv-2* gene. In one aspect, the plant of step a) comprising a *Cmv-2* gene is a plant of line 90-3588 or a progeny or ancestor of said plant. In one aspect, the present specification discloses a *Cucurbita* plant obtainable by any one of the methods above, wherein the plant comprises the *Cmv-2* gene. In one aspect, such plant is a *C. pepo* plant, in one aspect such plant is a *C. pepo* highly resistant to CMV. In one aspect, the present specification also discloses methods of producing a *Cucurbita* plant comprising a *Cmv-2* gene, for example comprising the steps set forth above.

The following paragraph is not part of the invention.

In one aspect, the present specification also discloses the use of a *Cmv-2* gene to confer resistance to CMV upon a *Cucurbita* plant susceptible thereto. In one aspect, the *Cmv-2* gene is used in the homozygous state in said *Cucurbita* plant. In one aspect, the present specification also discloses a method of conferring resistance to CMV to a *Cucurbita* plant susceptible thereto comprising: a) obtaining a plant comprising a *Cmv-2* gene; b) crossing it to a plant susceptible to CMV; c) obtaining plants of the cross of step b); d) selecting a plant of step c) which is resistant to CMV. In one aspect, the method further comprises: e) backcrossing a plant resulting from step d) with a *Cucurbita* plant, and f) selecting for a *Cucurbita* plant, which is resistant to CMV. In one aspect, the method further comprises obtaining an inbred *Cucurbita* plant, which is resistant to CMV, and, in one aspect, the method further comprises crossing said inbred *Cucurbita* plant to another *Cucurbita* plant to produce a hybrid *Cucurbita* plant, which is resistant to CMV. In one aspect, the plant of step a) comprising a *Cmv-2* gene is a plant of line 90-3588 or a progeny or ancestor of said plant. In one aspect, the present invention discloses a *Cucurbita* plant obtainable by any one of the methods above, wherein the plant is resistant to CMV. In one aspect, such plant is a *C. pepo* plant, in one aspect such plant is a *C. pepo* highly resistant to CMV.

In one embodiment, a *Cucurbita* plant of the present invention comprises a *Cmv-2* gene and a *Cmv-1* gene. For example, such a plant is obtained by crossing a plant of inbred line 90-3588 and a plant of hybrid 'Dividend' and selecting for a plant comprising both genes, for example using an allelic test as described herein.

Commercial *Cucurbita* are generally Fl hybrids produced from the cross of two parental lines (inbreds). The development of hybrids requires, in general, the development of homozygous inbred lines, the crossing of these lines, and the evaluation of the crosses. Pedigree breeding and recurrent selection breeding methods are used to develop inbred lines from breeding populations. Breeders combine the genetic backgrounds from two or more inbred lines or various other germplasm sources into breeding pools from which new inbred lines are developed by selfing and selection of desired phenotypes. The new inbreds are crossed with other inbred lines and the hybrids from these crosses are evaluated to determine which of those have commercial potential. Plant breeding and hybrid development are expensive and time-consuming processes.

The following paragraph is not part of the invention.

Accordingly, in one aspect, the present specification also discloses a method of producing hybrid seed comprising crossing a plant of the present disclosure, wherein said plant is an inbred line, with a plant of another inbred line; and harvesting hybrid seed resulting from the cross. In one aspect, both inbred lines in the cross are a plant of the present disclosure. In one aspect, the present specification also discloses hybrid seed produced by the method above, wherein said hybrid seed comprises a *Cmv-2* gene. In one aspect, a hybrid seed of the present has inbred line 90-3588 as a parent. In one aspect, a hybrid seed of the present disclosure is a seed of hybrid RSQ2193 (see Example 3 below).

Pedigree breeding starts with the crossing of two genotypes, each of which may have one or more desirable characteristics that is lacking in the other or which complements the other. If the two original parents do not provide all the desired characteristics, other sources can be included in the breeding population. In the pedigree method, superior plants are selfed and selected in successive generations. In the succeeding generations, the heterozygous condition gives way to homogeneous lines as a result of self-pollination and selection. Typically in the pedigree method of breeding five or more generations of self-pollination and selection is practiced: Fl to F2; F3 to F4; F4 to F5, etc.

A single cross hybrid results from the cross of two inbred lines, each of which has a genotype that complements the genotype of the other. The hybrid progeny of the first generation is designated Fl. In the development of commercial hybrids only the Fl hybrid plants are sought. Preferred Fl hybrids can be more vigorous than their inbred parents. This hybrid vigor, or heterosis, can be manifested in many polygenic traits, including increased vegetative growth and increased yield.

*Cucurbita* plants can be easily cross-pollinated. A trait is readily transferred from one *Cucurbita* plant to another *Cucurbita* plant to further obtain commercial lines. The introgression of a trait into a line is for example achieved by recurrent selection breeding, for example by backcrossing. In this case, the line (recurrent parent) is first crossed to a donor inbred (the non-recurrent parent) that carries the trait. The progeny of this cross is then mated back to the recurrent parent followed by selection in the resultant progeny for the trait. After three, preferably four, more preferably five or more generations of backcrosses with the recurrent parent with selection for the trait, the progeny is heterozygous for the locus harboring the resistance, but is like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376). Selection for the trait is carried out after each cross.

The traits are introgressed from another squash line, and the resulting plants have essentially all of the morphological and physiological characteristics of plants of inbred line 90-3588, in addition to the one or more traits introgressed into the inbred line. The invention also relates to seeds of an inbred line 90-3588 into which one or more traits have been introgressed and to plants of an inbred line 90-3588 into which one or more traits have been introgressed. The specification further relates to methods for producing a squash plant by crossing plants of an inbred line 90-3588 into which one or more traits have been introgressed with themselves or with another squash line. The invention also further relates to hybrid squash seeds and plants produced by crossing plants of an inbred line 90-3588 into which one or more traits have been introgressed with another squash line. Examples of traits transferred to inbred line 90-3588 include, but are not limited to, herbicide tolerance, resistance for bacterial, fungal, or viral disease, insect resistance.

In one embodiment, a *Cucurbita* plant of the present invention is male sterile. Male sterile lines do not produce viable pollen and cannot self-pollinate. By eliminating the pollen of one parental variety in a cross, a plant breeder is assured of obtaining hybrid seed of uniform quality. Therefore, the present invention discloses a male sterile *Cucurbita* plant, including seeds and materials of said plants and the progeny thereof. In one embodiment, a plant of the instant invention is a maintainer plant.

In one embodiment, a plant of the present invention is an inbred, a hybrid, or a dihaploid. In one embodiment, it is produced by pedigree breeding or by recurrent selection breeding. In one embodiment, a plant of the present invention has commercially acceptable agronomic characteristics.

*Cucurbita* plants can also be propagated vegetatively using methods well-known in the art, for example *in-vitro* plant tissue culture, rooting side shoot or protoplast fusion. In one embodiment, a method of vegetatively propagating a plant of the present invention comprises: a) collecting tissue of a plant of the present invention; b) cultivating said tissue to obtain proliferated shoots; c) rooting said proliferated shoots to obtain rooted plantlets; d) growing plants from said rooted plantlets; and harvesting seeds from said plants.

The following examples are intended to provide illustrations of the application of the present invention.

### EXAMPLES

### Example 1: Interspecific cross

A schematic description of the interspecific cross is provided below.

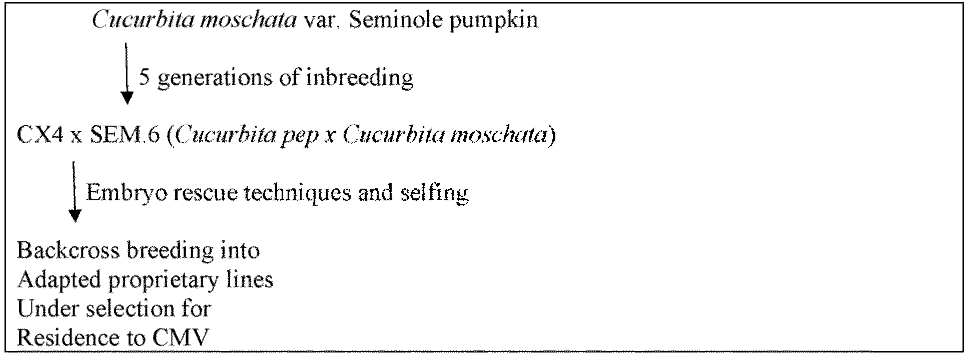

Detailed procedures are provided below:

### Cultivation of parental materials and artificial crossing

Plants of both parents were grown in the greenhouse under standard cultural practices. CX4 is a proprietary inbred line of the yellow straightneck type. *C. moschata* var. Seminole pumpkin was inbred for five cycles by artificial self-pollination to obtain SEM.6. Plants were grown in polyethylene pots (Vol = 10-15 L) filled with a soilless medium (peat moss, perlite, vermiculite, processed pine bark) and a balanced NPK (14:14:14), slow release fertilizer.

At flowering, female flowers of C. *pepo* were hand-pollinated with pollen from male flowers of *C. moschata.* The corolla of the male flower was removed with a twizer and the anthers were brushed on the stigma of the female flower for pollen transfer.

### Seed extraction and embryo explant

When the fruit set, 20 to 25 days after pollination, the fruit was harvested. The fruit was disinfected with ethanol and then cut in two halves along the vertical axis.

Seeds were extracted and dissected under a dissecting microscope. Embryos were separated from the seed using a forceps and a needle. The naked embryos were grown on V3 artificial medium (Tables 2-5) in a growth chamber under artificial conditions (t=25°C, 16 hours/day of light).

Seed dissection, embryo explant, and *in vitro* culture were performed under a laminar-flow sterile hood. All tools used for dissection and embryo transfer to the artificial medium were sterile.

### Seedling hardening

After roots form, the seedlings were transferred to polyethylene pots (4x4 cm) filled with peat. The seedlings were grown under artificial conditions in a growth chamber (t=25°C, 16 hours/day of light) and each pot was covered by a polyethylene bag, in order to maintain high humidity. The seedlings were watered daily as needed, with a water solution of the fungicide Previcur [2.5 mL/L], A hole (3-5 mm) was punched in the polyethylene bag after five days, in order to decrease humidity and acclimate the seedlings prior to removal of the bag.

### Plant cultivation and pollination

Once the roots had reached the borders of the pot, thus colonizing the medium, plants were transferred to the greenhouse or the open field and transplanted for self-pollination. Plants were grown under standard cultural practices and self-pollinated to create the first generation of *C. pepo* plants comprising the *Cmv-2* gene.

**Table 2. V3 medium used to culture the explanted embryos from the interspecific cross C. pepo X C. moschata.**

| Element | | Volume | |
|---|---|---|---|
| Murashige & Skoog, macroelements [10X] | | 100 mL | |
| Murashige & Skoog, microelements [100X] Morel | | 10 mL | |
| vitamines | | 2 mL | |
| Fe-EDTA | | 10 mL | |
| Sucrose | | 30 g | |
| Agar | | 8 g | |
| H₂O | up to 1 L | | |
| pH | 5.8±0.1 | | |

**Table 3. Preparation of 10X stock solution of Murashige & Skoog macroelements to be used in the preparation of the V3 culture medium.**

| Element | | Quantity (g) | |
|---|---|---|---|
| NH₄NO₃ | | 16.5' | |
| KNO₃ | | 19.0 | |
| CaCl₂ 2H₂O | | 4.4 | |
| MgSO₄ 7H₂KH₂PO₄ | | 3.7 | |
| | | 1.7 | |
| H₂O | up to 1 L | | |

**Table 4. Preparation of 100X stock solution of Murashige & Skoog microelements to be used in the preparation of the V3 culture medium.**

| Element | | Quantity (mg) | |
|---|---|---|---|
| H₃BO3 | | 620.0 | |
| MnSO₄ 1H₂O | | 1690.0 | |
| ZnSO₄ 7H₂O | | 860.0 | |
| KI | | 83.0 | |
| Na₂MoO₄ 2H₂O | | 25.0 | |
| CuSO₄ 5H₂O | | 2.5 | |
| CoCl₂ 6H₂O | | 2.5 | |
| H₂O | up to 1 L | | |

**Table 5. Preparation of 500X stock solution of Morel vitamins to be used in the preparation of the V3 culture medium.**

| Element | | Quantity (mg) |
|---|---|---|
| Inositol | | 5.0000 |
| Pyridoxine | | 0.0500 |
| Nicotinic acid | | 0.0500 |
| Thiamine | | 0.0500 |
| Calcium panthotenate | | 0.0500 |
| Biotin | | 0.0005 |
| H₂O | upto 100 mL | |

### Example 2: Disease testing

### Inoculum storage and increase

The Florida strain of cucumber mosaic virus (CMV) was used in the disease testing. This strain of CMV is highly pathogenic to squash and is maintained in permanent storage at Syngenta, Naples, Florida. Fresh infected squash leaves were stored in polyethylene bags at t=-72°C. In order to increase the amount of inoculum available, susceptible squash plants were inoculated, as described below, and infected mature leaves harvested and stored, when severe symptoms appeared (mosaic and mottling of leaves, and stunting of plants).

### Inoculation method

Squash leaves from cold storage were ground in a tissue blender in cool 0.02 M phosphate buffer (Table 6) at pH 7. 1±0.1. The inoculum solution was adjusted to a concentration of 1:6 (g of tissue:volume of buffer). The inoculum was maintained on ice for the duration of the inoculation process. Both cotyledons of plants at the first-true-leaf stage were inoculated by rubbing them with a gloved finger dipped into the inoculum solution. Gloves used were latex laboratory type and were changed every time the operator interrupted the inoculation procedure in order to perform other necessary steps (i.e.: moving flats).

### Growing conditions

Squash plants were sowed in 72-cell styrofoam speedling trays filled with a soilless medium (peat moss, perlite, vermiculite, processed pine bark). Trays were watered manually as needed and fertirrigated weekly with a soluble fertilizer (NPK 20:20:20). Inoculated trays were incubated in a growth chamber under artificial conditions (day t=85°C, night t=75°C, 16 hours of light/day). The growth chamber was organized with six shelves, three per layer, with upper and lower layers. Each shelf could accommodate six trays, side-by-side along their longer side. Each shelf had overhead fluorescent lights, adjustable in distance from the plants and lights were moved weekly during the growth cycle to maintain an average distance of 10 to 20 cm from the upper leaves of the plants, in order to prevent physiological stress to the plants and stretching and weakening of the seedlings.

### Disease assessment

Plants were rated for resistance to CMV two to three weeks after inoculation, once the susceptible checks show very severe mosaic and mottling of the leaves, and stunting of the plants. Single plants were counted by plot as resistant or susceptible.

Susceptible plants were identified when they showed the following symptoms of CMV infection at the seedling stage, depending on the severity of the infection:
- Malformation and drastic reduction in size of leaves.
- Development of a yellow mosaic pattern on leaves, followed by extensive necrosis under severe conditions.
- Under severe conditions, stunting of susceptible plants.

Resistant plants were identified as follows:
A. Highly resistant plants showed insignificant symptoms of CMV infection:
   - The first two true-leaves may show symptoms of CMV infection, as in susceptible plants.
   - New leaves grow without any mosaic symptom, nor distortion. The plants do not show symptoms thereafter.
B. Moderately resistant plants showed reduced symptoms of CMV infection:
   - Symptoms of CMV infection beyond the first two true-leaves, sometimes on most or all leaves.
   - Delayed appearance of symptoms of CMV infection and symptoms remaining throughout plant growth.
   - In some cases, moderately resistant plants may be overcome by the disease and may be rated as susceptible.
C. Immune plants showed no symptoms of CMV infection:
   - None of the leaves nor any other organ of the plant may show symptoms of CMV infection, such as mosaic or plant stunting.

Variability in the experiment was reduced by eliminating at inoculation all seedling that germinated more than three days later than the majority and by applying fungicides for prevention of fungal diseases. Pathogens that might infect plants in the growth chamber in Florida include gummy stem blight, caused by *Didymella bryoniae,* and powdery mildew, caused by *Sphaerotheca fuliginea.*

### Experimental design

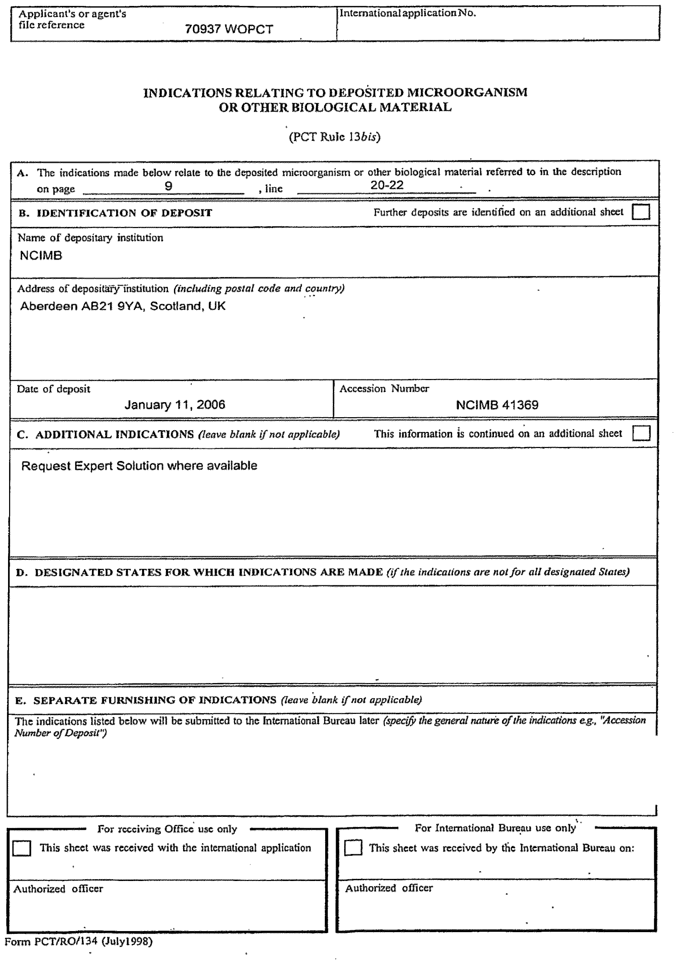

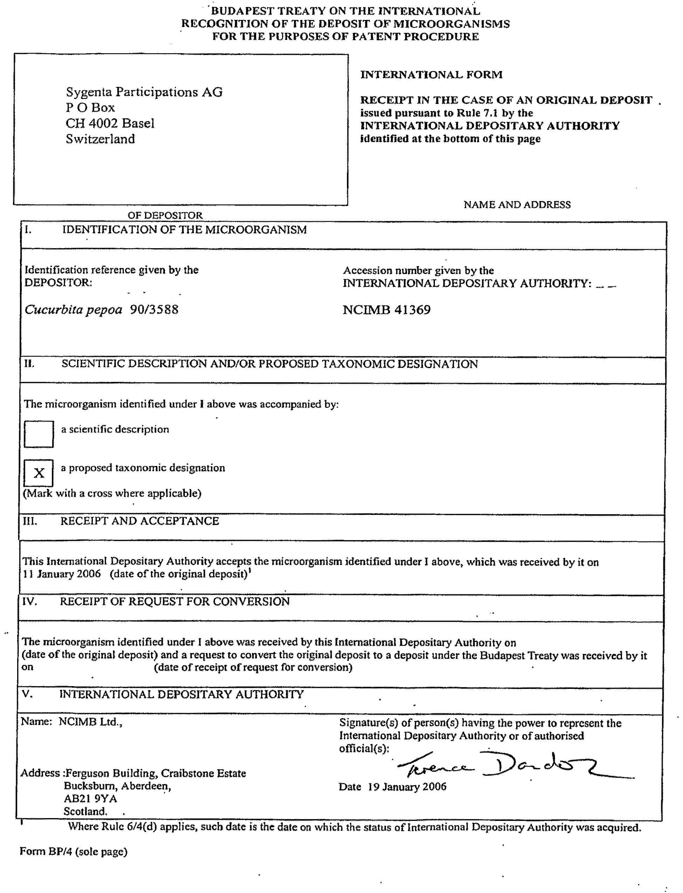

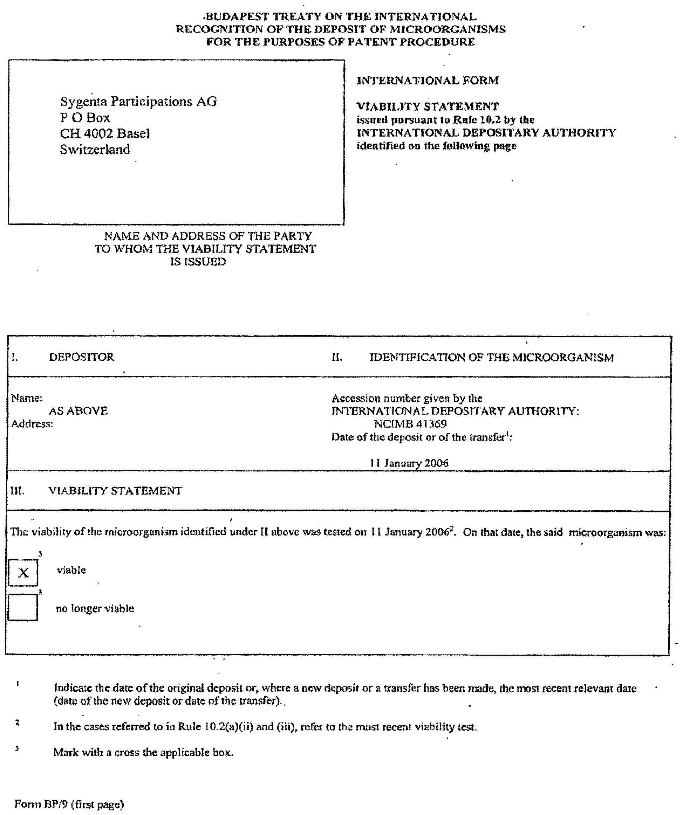

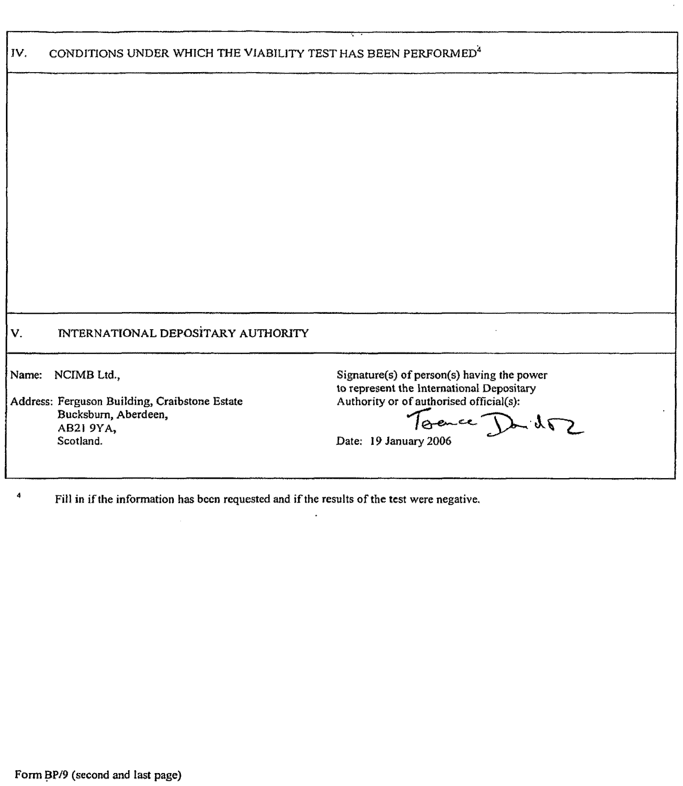

Cultigens (cultivars, experimental hybrids, and experimental inbreds) were planted in 30-plants plots (Figure 1). Cultigens were randomly assigned to plots and and plots (two per tray) were sequentially allocated to shelves in the growth chamber. Susceptible checks were planted in each tray with a frequency of 16.6% (1 plant every 6). Resistant checks were planted as 30-plants plots with a minimum frequency of one plot on each shelf of the growth chamber. When F2 and backcross segregating populations were screened, instead, singleplant plots were used.

**Table 6. Preparation of 10X stock solution of 0.02 M phosphate buffer to be used in inoculating CMV on squash seedlings.**

| Element | | Quantity (g) |
|---|---|---|
| KH₂PO₄ | | 3.4 |
| K2PO4 | | 8.7 |
| H₂O | up to 750 mL | |
| pH | 7.1±0.1 | |

| S | S | S | S | S | S | S | S | S | S | S | S |
|---|---|---|---|---|---|---|---|---|---|---|---|
| 25 | 26 | 27 | 28 | 29 | 30 | 25 | 26 | 27 | 28 | 29 | 30 |
| 19 | 20 | 21 | 22 | 23 | 24 | 19 | 20 | 21 | 22 | 23 | 24 |
| 13 | 14 | 15 | 16 | 17 | 18 | 13 | 14 | 15 | 16 | 17 | 18 |
| 07 | 08 | 09 | 10 | 11 | 12 | 07 | 08 | 09 | 10 | 11 | 12 |
| 01 | 02 | 03 | 04 | 05 | 06 | 01 | 02 | 03 | 04 | 05 | 06 |

Figure 1. Position of susceptible checks (S) and cultigens in each tray during screening experiments for resistance to CMV in squash.

### Example 3: Hybrid RSO2193

### Hybrid RSO2193

RSQ2193 is an experimental hybrid of summer squash *(Cucurbita pepo)* of yellow crookneck type. RSQ2193 is adapted to the Southeastern United States, where it produces high yields of high quality fruit (Table 7). RSQ2193 is similar in fruit type to 'Gentry' (a commercial variety of Syngenta Seeds, Inc.) but has better fruit quality, resistance to a wider spectrum of diseases, and a more vigorous plant. The parents of RSQ2193 are inbred lines 90-3588 and 90-3590.

Plants of RSQ2193 are open and upright to semi-prostrate. These plants may produce a few lateral branches. Leaves and stems are medium green and the peduncles have fewer and smaller spines than comparable cultivars such as 'Sunglo' (a commercial variety of Syngenta Seeds, Inc.) and 'Gentry'. The leaves are lobed, with denticulate flat margins and rounded apex. RSQ2193 carries orange male and female flowers, both fertile.

Fruit of RSQ2193 are yellow with a green peduncle and a semi-crooked neck. The fruit skin is very smooth, almost glabrous, with a very glossy finish. The blossom-end scar is small and flat to slightly protruted. The fruit are clubby, with a neck approximately 2/3 thinner than the main bulb of the fruit. The bulb is fairly elongated. The flesh quality at the marketable stage is comparable to 'Gentry', thus having a fine, dry, and soft texture.

Seeds of RSQ2193 are cream in color with a slightly darker rim around the edges. Seeds measure approximately 14 mm in length, 8 mm in width, and 3 mm in thickness. Seed coat is smooth, dull, and has curved and rounded margins; Seeds separate easily from the pulp at seed harvest.

RSQ2193 is highly resistant to cucumber mosaic virus (CMV) (Table 8), and powdery mildew caused by *Sphaerotheca fuliginea.* The resistance to CMV present in RSQ2193 is from *C. moschata* var. Seminole pumpkin, and has been transferred to this hybrid through interspecific cross as described in the instant application. RSQ2193 is homozygous for the *Cmv-2* gene.

**Table 7. Yield data collected in grower's fields in Georgia in the Summer of 2005 for 10 RSQ2193. Destiny III and Prelude II are commercial varieties of Seminis, Inc.**

| | | Yield (Boxes/acre) | | |
|---|---|---|---|---|
| Location | Cultivar | No.1^{a} | No.2^{a} | Total |
| 1 | RSQ2193 | 456 | 572 | 1028 |
| 1 | Destiny III | 391 | 526 | 917 |
| 2 | RSQ2193 | 334 | 467 | 801 |
| 2 | Gentry | 362 | 517 | 879 |
| 2 | Prelude II | 301 | 482 | 783 |
| 3 | RSQ2193 | 371 | 457 | 828 |
| 3 | Gentry | 411 | 476 | 887 |
| 3 | Prelude II | 380 | 443 | 823 |
| 4 | RSQ2193 | 385 | 448 | 833 |
| 4 | Gentry | 393 | 521 | 914 |
| 4 | Prelude II | 349 | 466 | 815 |
| 4 | Destiny III | 362 | 393 | 755 |
| 5 | RSQ2193 | 483 | 581 | 1064 |
| 5 | Prelude II | 467 | 564 | 1031 |

| | | | | |
|---|---|---|---|---|
| a No. 1, #2 = Fruit quality categories, as determined by the 'USDA-ARS Standards for Grades of Summer Squash' (Effective January 6, 1984 (Reprinted - January 1997) | | | | |

### Inbred line 90-3588

Inbred line 90-3588 is a proprietary inbred line of summer squash *(C. pepo)* of yellow crookneck type. Line 90-3588 is a parent of hybrid RSQ2193 described above. Line 90-3588 is similar in fruit type to 'Gentry' and comprises in homozygous condition the *Cmv-2* gene for resistance to cucumber mosaic virus.

Leaves of 90-3588 are entire, rounded, and medium green. Fruit of line 90-3588 are yellow with a green peduncle and a crooked neck. Seeds of line 90-3588 are cream in color with a slightly darker rim around the edges. Seeds measure approximately 13 mm in length, 8 mm in width, and 3 mm in thickness. Seed coat is smooth, dull, and has curved and rounded margins. Seeds separate easily from the pulp at seed harvest.

The resistance to CMV present in the genetics of line 90-3588 is from *C. moschata* var. Seminole pumpkin, and has been transferred to this inbred line through interspecific cross as described in the instant application.

**Table 8. Resistance of RSQ2193 and parental inbreds to CMV in the screening performed in 2005 in Naples, Florida.**

| | | No. Resistant / No. Inoculated | | | | | |
|---|---|---|---|---|---|---|---|
| | | Rep 1 | Rep 2 | Rep 3 | Rep 4 | Rep 5 | Rep 6 |
| RSQ2193 | | | | | | | |
| | Female Parent | 28/28 | 27/27 | 28/28 | 27/27 | 30/30 | 29/29 |
| | Male Parent | 24/24 | 25/25 | 21/21 | 25/25 | 26/26 | 22/22 |
| | Hybrid | 29/29 | 30/30 | 29/29 | 29/29 | 28/28 | 30/30 |

| Checks | | | | | | | |
|---|---|---|---|---|---|---|---|
| | Resistant^{a} | 36/36 | 36/36 | 36/36 | 36/36 | 36/36 | 36/36 |
| | Susceptible | 0/26 | 0/30 | 0/26 | 0/29 | 0/29 | 0/29 |

| | | | | | | | |
|---|---|---|---|---|---|---|---|
| a *C. moschata* var. Seminole pumpkin, original source of resistance used to introgress resistance into RSQ2193 | | | | | | | |

Example 4: Disease testing of *C. moschata* cv. Seminole Pumpkin and Nigerian Local *C. moschata* cv. Seminole Pumpkin and *C. moschata* cv. Nigerian Local were tested for resistance to CMV as described in Example 2 above. Nigerian Local is a winter squash, species *Cucurbita moschata,* described in Brown, 2003 (Euphytica 129: 253-258).

Plants of *C. moschata* cv. Seminole Pumpkin were rated as immune to CMV and did not show any symptom of the disease. Plants of *C. moschata* cv. Nigerian Local were rated as moderately resistant to CMV, but showed symptoms of CMV infection on all plants tested. The symptoms in *C. moschata* cv. Nigerian Local were limited to mosaic on the leaves and none of the tested plants was stunted (Table 9).

**Table 9. Level of resistance to CMV in C. moschata cv. Seminole Pumpkin and Nigerian Local in a screening in Naples, Florida.**

| Six repetitions were carried out and plants were rated for resistance to CMV. The number of plants rated in each of the repetitions is indicated. The susceptible check used was an inbred line susceptible to all diseases, internally named "Universal susceptible" 90-3871. | | | | | | | |
|---|---|---|---|---|---|---|---|
| | | Rep 1 | Rep 2 | Rep 3 | Rep 4 | Rep 5 | Rep 6 |
| *C. moschata* cv. Nigerian Local | | | | | | | |
| | Immune | 0 | 0 | 0 | 0 | 0 | 0 |
| | Moderately resistant | 24 | 30 | 30 | 26 | 29 | 30 |
| | Susceptible | 0 | 0 | 0 | 0 | 0 | 0 |
| | Total tested | 24 | 30 | 30 | 26 | 29 | 30 |

| *C. moschata* cv. Seminole Pumpkin | | | | | | | |
|---|---|---|---|---|---|---|---|
| | Immune | 29 | 30 | 30 | 30 | 29 | 24 |
| | Moderately resistant | 0 | 0 | 0 | 0 | 0 | 0 |
| | Susceptible | 0 | 0 | 0 | 0 | 0 | 0 |
| | Total tested | 29 | 30 | 30 | 30 | 29 | 24 |

| Susceptible Check | | | | | | | |
|---|---|---|---|---|---|---|---|
| | Immune | 0 | 0 | 0 | 0 | 0 | 0 |
| | Moderately resistant | 0 | 0 | 0 | 0 | 0 | 0 |
| | Susceptible | 29 | 30 | 30 | 30 | 29 | 24 |
| | Total tested | 29 | 30 | 30 | 30 | 29 | 24 |

### Example 5: Allelic test

An allelic test was carried out between plants of a breeding line of *C. pepo* comprising a *Cmv-2* gene and plants of a breeding line of *C. pepo* comprising a *Cmv-1* gene.

In a disease testing as described in Example 2 above, the disease was sometimes capable of overcoming the resistance conferred by the *Cmv-1* gene leading to difficulties in rating plants comprising a *Cmv-1* gene. Such plants may thus be rated resistant or susceptible. By contrast, the *Cmv-2* gene confers a higher and more stable level of resistance, leading to a clear rating as resistant. In addition, the phenotypic manifestation of symptoms on resistant plants was different. Individuals plants containing a *Cmv-1* gene but not a *Cmv-2* gene may have a resistant reaction on the first two leaves to develop, following inoculation, while the subsequent leaves may look susceptible and the entire plant may eventually stunt and collapse at a later time. Plants homozygous or heterozygous for a *Cmv-2* gene, instead, may show initial symptoms on the inoculated leaves. Plants would recover and from the third true-leaf on they would not show any more symptom of CMV infection.

Based on this differential expression of resistance, and the stronger effect of the *Cmv-*2 gene, the expected phenotypic frequencies (Table 10) may not be easily observed in artificial tests for resistance. The higher level of resistance conferred by the *Cmv-2* gene was indicated by the phenotypic rating of the Fl hybrid heterozygous at both loci (Table 11, Entry 5) and the inbred line homozygous dominant for the *Cmv-2* gene and homozygous recessive at the *Cmv- 1* locus (Table 11, Entry 2).

In the F2 populations derived from a cross of an inbred line homozygous dominant for a *Cmv-1* gene, and an inbred line homozygous dominant for a *Cmv-2* gene, plants susceptible to CMV were recovered (Table 11). This demonstrates that *Cmv-1* and *Cmv-2* in the populations of *C. pepo.used* are two different genes.

The segregation ratio recorded of 206:48 (resistant:susceptible) plants deviated the expected 15:1 segregation ratio for two single dominant genes. However, the Fl hybrid and the inbred lines carrying a *Cmv-1* gene (in heterozygous and homozygous condition, respectively) also contained individuals that were rated as susceptible in the test.

In conclusion, the allelic test indicates that the *Cmv-2* gene is located at a different chromosomal location than the *Cmv-1* gene. The *Cmv-2* gene also confers higher level of resistance to CMV upon a *C. pepo* plant than the *Cmv-1* gene. The unbalanced segregation ratio recorded in the F2 population tested can be explained by the mild and unstable resistance conferred by the *Cmv-1* gene, in absence of the *Cmv-2* gene. Individuals that were homozygous recessive at the *Cmv-2* locus would have received only mild and unstable resistance from the *Cmv-1* gene, thus some times being rated as susceptible.

The population should have included 32 individuals heterozygous for the *Cmv-I* gene and homozygous recessive for the *Cmv-2* gene, 16 individuals homozygous dominant for the *Cmv-1* gene and homozygous recessive for the *Cmv-2* gene, and 16 individuals double recessive (phenotypic expectations base on Mendel's inheritance rules and equal effects of two unlinked genes). Because of the instability or mild expression of resistance due to the *Cmv-1* gene, the phenotype of the double recessive individuals may have been confounded with that of several individuals pertaining to the other two genotypic classes. Furthermore, this hypothesis is strongly supported by the evidence that the inbred homozygous dominant for the *Cmv-1 gene,* and not carrying the *Cmv-2* gene, showed susceptible individuals at a high frequency (0.42).

**Table 10. Segregation of the Cmv-1 and Cmv-2 genes in Cucurbita pepo, based on a two-gene independent inheritance model (Mendel).**

| | Genotype^{a} | | | Phenotype^{b} | |
|---|---|---|---|---|---|
| | *Cmv-1* | *Cmv-2* | Freq^{c} | Expected^{d} | Possible^{e} |
| 1 | R | R | 1/16 | R | R |
| 2 | R | H | 2/16 | R | R |
| 3 | R | S | 1/16 | R | R/S |
| 4 | H | R | 2/16 | R | R |
| 5 | H | H | 4/16 | R | R |
| 6 | H | S | 2/16 | R | R/S |
| 7 | S | R | 1/16 | R | R |
| 8 | S | H | 2/16 | R | R |
| 9 | S | S | 1/16 | S | S |

| | | | | | |
|---|---|---|---|---|---|
| a Genotype at the *Cmv-1* and *Cmv-2* loci. R = homozygous dominant; S = homozygous recessive; H = heterozygous; Seg = segregating according to Mendel's laws. b Phenotypic data for resistance to CMV artificial inoculation. R = resistant; S = susceptible. c Expected genotypic frequency. d Expected phenotype, based on Mendel's inheritance rules. e Possible phenotype, due to the mild and unstable resistance conferred by the *Cmv-1* gene. | | | | | |

**Table 11. Segregation of the Cmv-1 and Cmv-2 genes in Cucurbita pepo, as measured in Naples, Florida.**

| | Genotype^{a} | | | | Phenotype^{b} | | |
|---|---|---|---|---|---|---|---|
| | | *Cmv-1* | *Cmv-2* | | Res | Sus | Dead |
| | Gen^{c} | | | Tot^{d} | | | |
| Entry 1 | IB | R | S | 21 | 12 | 8 | 1 |
| Entry 2 | IB | S | R | 119 | 118 | 1 | 0 |
| Entry 3 | Fl | H | S | 29 | 21 | 6 | 2 |
| Entry 4 | Fl | S | H | - | | | - |
| Entry 5 | Fl | H | H | 8 | 8 | 0 | 0 |
| Entry 6 | F2 | Seg | Seg | 255 | 206 | 48 | 1 |

| | | | | | | | |
|---|---|---|---|---|---|---|---|
| a Genotype at the *Cmv-]* and *Cmv-2* loci. R = homozygous dominant; S = homozygous recessive; H = heterozygous; Seg = segregating according to Mendel's laws. b Phenotypic data for resistance to CMV artificial inoculation. Res = number of resistant plants; Sus = number of susceptible plants; Dead = number of dead plants. c Generation. IB = inbred; Fl, F2 = Fl and F2 hybrids. d Total number of plants tested. | | | | | | | |

## Claims

1. A *Cucurbita* plant comprising a *Cmv-2* gene conferring Cucumber Mosaic Virus (CMV) resistance to said *Cucurbita* plant, wherein said *Cmv-2* gene is obtainable from a plant of *C. moschata* cv. Seminole pumpkin, wherein, when said *Cucurbita* plant is crossed with a plant of inbred line 90-3588, 100% of F1 plants resulting from said cross are resistant to CMV, and 100% of said F1 plants produce 100% of F2 progeny plants resistant to CMV, wherein said *Cucurbita* plant is homozygous for a *Cmv-2* gene, wherein said plant is not a plant of *C. moschata* cv. Seminole pumpkin, wherein said plant is male sterile, wherein resistance to CMV is monogenic and wherein said *Cmv-2* gene is obtainable from a plant of line 90-3588, representative seed of which being deposited under accession number NCIMB 41369.

2. The *Cucurbita* plant of claim 1, wherein said plant is a *C. pepo* plant, a *C. maxima* plant or a *C. mixta* plant.

3. Seed of a *Cucurbita* plant according to any one of claims 1 to 2; wherein said seed comprises a *cmv-2* gene conferring CMV resistance to said *Cucurbita* plant, and wherein said *Cmv-2* gene is obtainable from a plant of line 90-3588, representative seed of which being deposited under accession number NCIMB 41369.

4. A method of vegetatively propagating a *Cucurbita* plant comprising:
a) collecting a tissue of a *Cucurbita* plant comprising a *Cmv-2* gene conferring Cucumber Mosaic Virus (CMV) resistance to said *Cucurbita* plant, wherein, when said *Cucurbita* plant is crossed with a plant of inbred line 90-3588, 100% of F1 plants resulting from said cross are resistant to CMV, and 100% of said F1 plants produce 100% of F2 progeny plants resistant to CMV, when said *Cucurbita* plant is homozygous for a *Cmv-2* gene, wherein said plant is not a plant of *C. moschata* cv. Seminole Pumpkin, wherein said plant is an inbred line, and wherein said *Cmv-2* gene is obtainable from a plant of line 90-3588, representative seed of which being deposited under accession number NCIMB 41369;
b) cultivating said tissue to obtain proliferated shoots;
c) rooting said proliferated shoots to obtain rooted plantlets.

## Patentansprüche

1. *Cucurbita*-Pflanze, umfassend ein *Cmv-2*-Gen, das der *Cucurbita*-Pflanze Resistenz gegen CMV (Cucumber Mosaic Virus) verleiht, wobei das *Cmv-2*-Gen aus einer Pflanze des Kürbisses *C. moschata* cv. Seminole erhältlich ist, wobei beim Kreuzen der *Cucurbita*-Pflanze mit einer Pflanze der Inzuchtlinie 90-3588 100 % der aus der Kreuzung erhaltenen F1-Pflanzen gegen CMV resistent sind und 100 % der F1-Pflanzen 100 % gegen CMV resistente F2-Nachkommenpflanzen produzieren, wobei die *Cucurbita-*Pflanze homozygot für ein *Cmv-2*-Gen ist, wobei es sich bei der Pflanze nicht um eine Pflanze des Kürbisses *C. moschata* cv. Seminole handelt, wobei die Pflanze männlich steril ist, wobei Resistenz gegen CMV monogen ist und wobei das *Cmv-2*-Gen aus einer Pflanze der Linie 90-3588 erhältlich ist, von der repräsentative Samen unter Zugangsnummer NCIMB 41369 hinterlegt sind.

2. *Cucurbita-Pflanze* nach Anspruch 1, wobei es sich bei der Pflanze um eine *C. pepo*-Pflanze, eine *C. maxima-*Pflanze oder eine *C.* mixta-Pflanze handelt.

3. Samen einer *Cucurbita-Pflanze* gemäß einem der Ansprüche 1 bis 2; wobei der Samen ein *cmv-2*-Gen, das der *Cucurbita*-Pflanze Resistenz gegen CMV verleiht, umfasst und wobei das *Cmv-2*-Gen aus einer Pflanze der Linie 90-3588 erhältlich ist, von der repräsentative Samen unter Zugangsnummer NCIMB 41369 hinterlegt sind.

4. Verfahren zur vegetativen Vermehrung einer *Cucurbita*-Pflanze, umfassend:
a) Entnehmen eines Gewebes einer *Cucurbita*-Pflanze, die ein *Cmv-2*-Gen umfasst, das der *Cucurbita*-Pflanze Resistenz gegen CMV (Cucumber Mosaic Virus) verleiht, wobei beim Kreuzen der *Cucurbita*-Pflanze mit einer Pflanze der Inzuchtlinie 90-3588 100 % der aus der Kreuzung erhaltenen F1-Pflanzen gegen CMV resistent sind und 100 % der F1-Pflanzen 100 % gegen CMV resistente F2-Nachkommenpflanzen produzieren, wenn die *Cucurbita-*Pflanze homozygot für ein *Cmv-2*-Gen ist, wobei es sich bei der Pflanze nicht um eine Pflanze des Kürbisses *C. moschata* cv. Seminole handelt, wobei es sich bei der Pflanze um eine Inzuchtlinie handelt und wobei das *Cmv-2*-Gen aus einer Pflanze der Linie 903588 erhältlich ist, von der repräsentative Samen unter Zugangsnummer NCIMB 41369 hinterlegt sind;
b) Kultivieren des Gewebes zur Gewinnung vermehrter Ausläufer;
c) Bewurzeln der vermehrten Ausläufer zur Gewinnung bewurzelter Setzlinge.

## Revendications

1. Végétal de *Cucurbita* comprenant un gène *Cmv-2* conférant une résistance au virus de la mosaïque du concombre (CMV) audit végétal de *Cucurbita,* ledit gène *Cmv-2* pouvant être obtenu à partir d'un végétal de *C. moschata* cv. citrouille séminole, lorsque ledit végétal de *Cucurbita* est croisé avec un végétal de lignée consanguine 90-3588, 100 % de végétaux F1 résultant dudit croisement étant résistants à CMV, et 100 % desdits végétaux F1 produisant 100 % de végétaux descendants F2 résistants à CMV, ledit végétal de *Cucurbita* étant homozygote pour un gène *Cmv-2,* ledit végétal n'étant pas un végétal de *C. moschata* cv. citrouille séminole, ledit végétal étant un mâle stérile, la résistance à CMV étant monogénique et ledit gène *Cmv-2* pouvant être obtenu à partir d'un végétal de lignée 90-3588, une graine représentative duquel étant déposée sous le numéro d'accès NCIMB 41369.

2. Végétal de *Cucurbita* selon la revendication 1, ledit végétal étant un végétal de *C. pepo,* un végétal de *C. maxima* ou un végétal de *C. mixta.*

3. Graine d'un végétal de *Cucurbita* selon l'une quelconque des revendications 1 à 2 ; ladite graine comprenant un gène *Cmv-2* conférant une résistance à CMV audit végétal de *Cucurbita,* ledit gène *Cmv-2* pouvant être obtenu à partir d'un végétal de lignée 90-3588, une graine représentative duquel étant déposée sous le numéro d'accès NCIMB 41369.

4. Procédé de propagation de manière végétative d'un végétal de *Cucurbita* comprenant :
a) la collecte d'un tissu d'un végétal de *Cucurbita* comprenant un gène *Cmv-2* conférant une résistance au virus de la mosaïque du concombre (CMV) audit végétal de *Cucurbita,* lorsque ledit végétal de *Cucurbita* est croisé avec un végétal de lignée consanguine 90-3588, 100 % de végétaux F1 résultant dudit croisement étant résistants à CMV, et 100 % desdits végétaux F1 produisant 100 % de végétaux descendants F2 résistants à CMV, lorsque ledit végétal de *Cucurbita* est homozygote pour un gène *Cmv-2,* ledit végétal n'étant pas un végétal de *C. moschata* cv. citrouille séminole, ledit végétal étant une lignée consanguine, et ledit gène *Cmv-2* pouvant être obtenu à partir d'un végétal de lignée 90-3588, une graine représentative duquel étant déposée sous le numéro d'accès NCIMB 41369 ;
b) la culture dudit tissu pour obtenir des pousses proliférées ;
c) l'enracinement desdites pousses proliférées pour obtenir des plantules enracinées.
